# EUROPEAN PATENT APPLICATION

(11) **EP 4 349 199 A2**
(43) Date of publication of application: **10.04.2024**
(21) Application number: 24159339.1
(22) Date of filing: 30.10.2019
(51) Int. Cl.: A24F 40/90

(54) **POWER SUPPLY UNIT FOR AEROSOL INHALER AND CONTROL METHOD OF THE SAME**

(30) Priority: 31.10.2018 JP 2018204702
(62) Divisional of application: 19206192.7
(71) Applicant: Japan Tobacco, Inc., Tokyo, 105-6927 (JP)
(72) Inventor: Yamada, Manabu, Tokyo, 130-8603 (JP); Akao, Takeshi, Tokyo, 130-8603 (JP); Fujita, Hajime, Tokyo, 130-8603 (JP)
(74) Representative: Hoffmann Eitle

(57) **Abstract**

A power supply unit for an aerosol inhaler includes: a power supply unit; a charging terminal electrically connected to an external power supply charging the power supply; a discharging terminal electrically connected to a load generating an aerosol from an aerosol source; and a control unit detecting a request for aerosol generation and controlling discharging of the power supply through the discharging terminal and charging of the power supply through the charging terminal. The external power supply can be electrically connected to the charging terminal in a state where discharging of the power supply through the discharging terminal is possible. The control unit performs control such that the charging and the discharging are not performed at the same time in a case where the control unit detects the request in a state where the charging terminal and the external power supply are electrically connected.

## Description

### TECHNICAL FIELD

The present invention relates to a power supply unit for an aerosol inhaler, and a control method and control program of the power supply unit.

### BACKGROUND ART

Patent Literature 1 discloses a non-combustion type flavor inhaler, which has an atomizing unit having a load for atomizing an aerosol source without combustion, and a power supply unit including a power supply for supplying power to the load. This flavor inhaler has a connection part configured at one end part of the power supply unit such that the load and a charger can be alternatively connected thereto.

To the connection part of the power supply unit, either the load of the atomizing unit or the charger can be exclusively connected, and the charger and the load cannot be connected at the same time.

Patent Literature 1: WO 2018/163261

Here, it has been studied to configure the power supply unit such that charging is possible without detaching the atomizing unit from the aerosol inhaler such as a flavor inhaler in consideration with user convenience. However, if separately providing a discharging terminal and a charging terminal on the power supply unit, it structurally becomes possible to perform discharging of the power supply through the discharging terminal and charging of the power supply through the charging terminal at the same time.

In power supplies useable in aerosol inhalers, when discharging and charging are performed at the same time, deterioration of power supplies may accelerate. For this reason, it is required to effectively suppress deterioration of power supplies while considering user convenience.

An object of the present invention is to provide a power supply unit for an aerosol inhaler, and a control method and control program of the power supply unit, capable of suppressing deterioration of a power supply.

### SUMMARY OF INVENTION

According to an aspect of the invention, there is provided a power supply unit for an aerosol inhaler, the power supply unit comprising: a power supply unit; a charging terminal that is able to be electrically connected to an external power supply capable of charging the power supply; a discharging terminal that is able to be electrically connected to a load for generating an aerosol from an aerosol source and is separate from the charging terminal; and a control unit that is configured to detect a request for aerosol generation and controls discharging of the power supply through the discharging terminal and charging of the power supply through the charging terminal, wherein the power supply unit is configured such that the external power supply can be electrically connected to the charging terminal in a state where discharging of the power supply through the discharging terminal is possible, and the control unit performs control such that the charging and the discharging are not performed at the same time in a case where the control unit detects the request in a state where the charging terminal and the external power supply are electrically connected.

### BRIEF DESCRIPTION OF DRAWINGS

Fig. 1 is a perspective view of an aerosol inhaler equipped with a power supply unit of an embodiment of the present invention.
Fig. 2 is another perspective view of the aerosol inhaler of Fig. 1.
Fig. 3 is a cross-sectional view of the aerosol inhaler of Fig. 1.
Fig. 4 is a perspective view of the power supply unit.
Fig. 5 is a block diagram of the power supply unit.
Fig. 6 is an electric circuit diagram of the power supply unit.
Fig. 7 is a view illustrating the relation between the amount of aerosol generation and a control signal.
Fig. 8 is an explanatory view for explaining charge/discharge control of a first example.
Fig. 9 is an explanatory view for explaining charge/discharge control of a second example.
Fig. 10 is an explanatory view for explaining charge/discharge control of a third example.
Fig. 11 is an explanatory view for explaining charge/discharge control of a fourth example.
Fig. 12 is an electric circuit diagram of a power supply unit of a modification.

### DESCRIPTION OF EMBODIMENTS

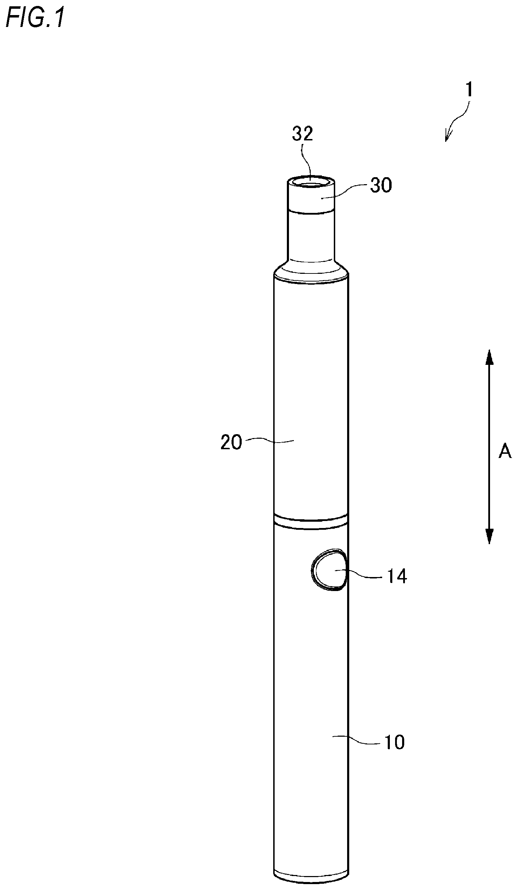

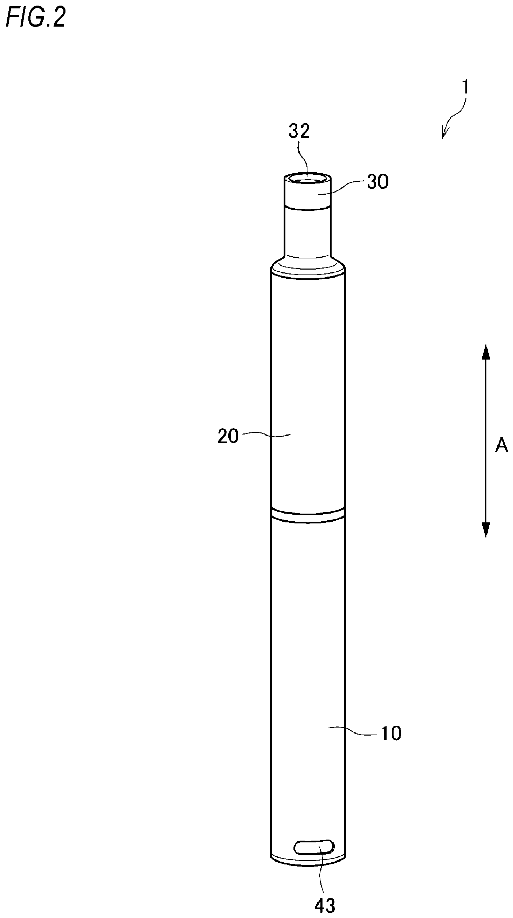

Hereinafter, a power supply unit for an aerosol inhaler according to an embodiment of the present invention will be described. First of all, the aerosol inhaler equipped with the power supply unit will be described with reference to Fig. 1 to Fig. 3.

### (AEROSOL INHALER)

An aerosol inhaler 1 is a device for inhaling a flavor without combustion, and has a rod shape extending along a certain direction (hereinafter, referred to as the longitudinal direction A). The aerosol inhaler 1 includes a power supply unit 10, a first cartridge 20, and a second cartridge 30 which are arranged in the order along the longitudinal direction A. The first cartridge 20 can be attached to and detached from the power supply unit 10, and the second cartridge 30 can be attached to and detached from the first cartridge 20. In other words, the first cartridge 20 and the second cartridge 30 can be individually replaced.

### (POWER SUPPLY UNIT)

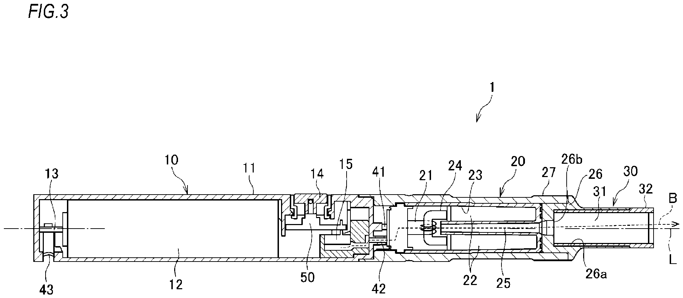

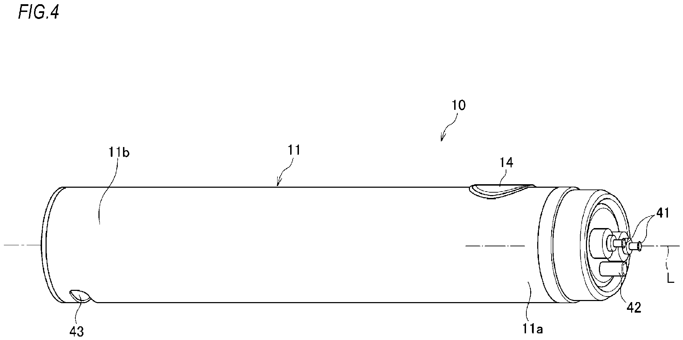

The power supply unit 10 of the present embodiment includes a power supply 12, a charger 13, a control unit 50, various sensors, and so on in a cylindrical power supply unit case 11, as shown in Fig. 3 and Fig. 4. The power supply 12 is a chargeable secondary battery, an electric double-layer capacitor, or the like, and is preferably a lithium-ion battery.

On a top part 1 1a of the power supply unit case 11 positioned on one end side in the longitudinal direction A (the first cartridge (20) side), a discharging terminal 41 is provided. The discharging terminal 41 is provided so as to protrude from the top surface of the top part 11a toward the first cartridge 20, and is configured to be able to be electrically connected to a load 21 of the first cartridge 20.

Further, on a part of the top surface of the top part 1 1a in the vicinity of the discharging terminal 41, an air supply part 42 for supplying air to the load 21 of the first cartridge 20 is provided.

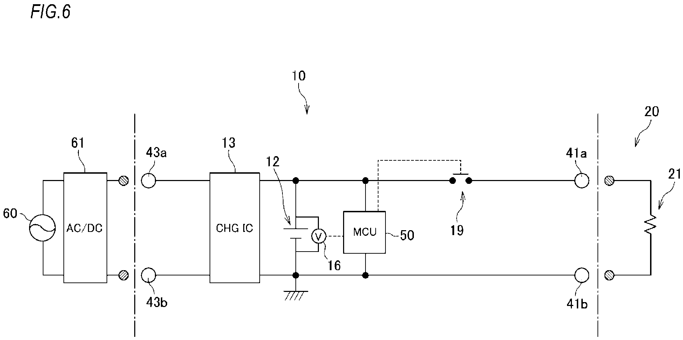

On a bottom part 11b of the power supply unit 10 positioned on the other end side in the longitudinal direction (the opposite side to the first cartridge 20), a charging terminal 43 able to be electrically connected to an external power supply 60 (see Fig. 6) capable of charging the power supply 12 is provided. The charging terminal 43 is provided on the side surface of the bottom part 11b, such that at least one of USB terminals, micro USB terminals, and Lightning terminals can be connected thereto.

However, the charging terminal 43 may be a power receiving part able to receive power from the external power supply 60 in a non-contact manner. In this case, the charging terminal 43 (the power receiving part) may be composed of a power receiving coil. The wireless power transfer system may be an electromagnetic induction type, or may be a magnetic resonance type. Also, the charging terminal 43 may be a power receiving part able to receive power from the external power supply 60 without any contact point. As another example, the charging terminal 43 may be configured such that at least one of USB terminals, micro USB terminals, and Lightning terminals can be connected thereto and the above-mentioned power receiving part is included therein.

In other words, the discharging terminal 41 and the charging terminal 43 are separately configured, and are disposed apart in the longitudinal direction A. Therefore, the power supply unit 10 is configured such that it is possible to electrically connect the external power supply 60 to the charging terminal 43 in the state where discharging of the power supply 12 through the discharging terminal 41 is possible. More specifically, in the power supply unit 10, in the state where the charging terminal 43 and the external power supply 60 are electrically connected, in the case where an aerosol generation request is detected, charging and discharging of the power supply 12 are prohibited from being performed at the same time.

Also, on the side surface of the top part 11a of the power supply unit case 11, an operation unit 14 which the user can operate is provided so as to face the opposite side to the charging terminal 43. More specifically, the operation unit 14 and the charging terminal 43 are symmetric with respect to the point of intersection of a straight line connecting the operation unit 14 and the charging terminal 43 and the center line L of the power supply unit 10 in the longitudinal direction A. The operation unit 14 is composed of a button type switch, a touch panel, or the like, and is used to perform various processes such as a process of activating and shutting off the control unit 50 and various sensors according to user's intention to use. In the vicinity of the operation unit 14, the control unit 50 and an inhalation sensor 15 for detecting a puff action are provided.

The charger 13 is disposed close to the charging terminal 43, and controls charging power from the charging terminal 43 to be input to the power supply 12. The charger 13 includes a converter for converting direct current, which is applied from an inverter 61 or the like provided for converting alternating current into direct current on a charging cable which is connected to the charging terminal 43, into direct current having a different magnitude, a voltmeter, an ammeter, a processor, and so on.

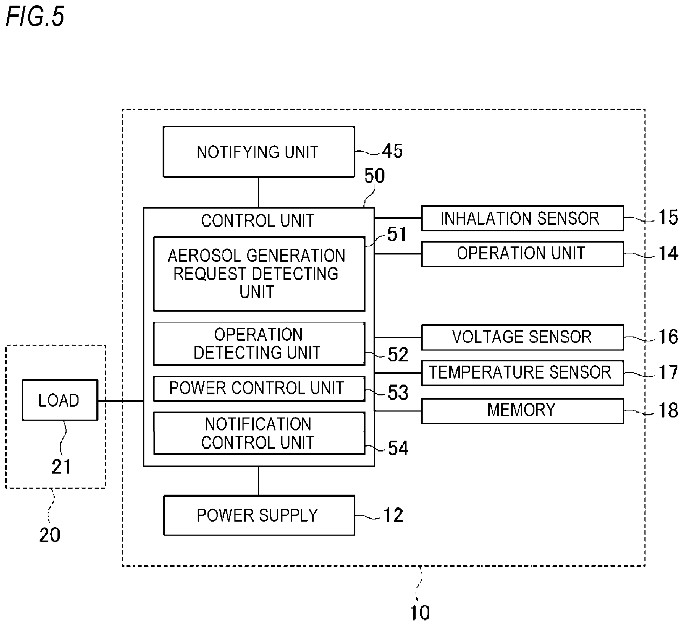

The control unit 50 is connected to various sensor devices, such as the inhalation sensor 15 for detecting a puff (inhaling) action, a voltage sensor 16 for measuring the voltage of the power supply 12, and a temperature sensor 17, the operation unit 14, and a memory 18 for storing the number of puff actions, the time for which power has been applied to the load 21, and so on, as shown in Fig. 5, and performs a variety of control on the aerosol inhaler 1. The inhalation sensor 15 may be configured with a capacitor microphone, a pressure sensor, or the like. The control unit 50 is specifically a processor (a computer). More specifically, the structure of this processor is an electric circuit configured by combining circuit elements such as semiconductor elements. The details of the control unit 50 will be described below.

Also, in the power supply unit case 11, an air intake (not shown in the drawings) for taking in air is formed. The air intake may be formed around the operation unit 14, or may be formed around the charging terminal 43.

### (FIRST CARTRIDGE)

As shown in Fig. 3, the first cartridge 20 includes a reservoir 23 for storing an aerosol source 22, the electric load 21 for atomizing the aerosol source 22, a wick 24 for drawing the aerosol source from the reservoir 23 toward the load 21, an aerosol channel 25 for an aerosol generated by atomizing the aerosol source 22 to flow toward the second cartridge 30, an end cap 26 for storing a part of the second cartridge 30.

The reservoir 23 is formed so as to surround the aerosol channel 25, and holds the aerosol source 22. In the reservoir 23, a porous member such as a resin web or cotton may be stored, and the porous member may be impregnated with the aerosol source 22. The aerosol source 22 includes a liquid such as glycerin, propylene glycol, or water.

The wick 24 is a liquid holding member for drawing the aerosol source 22 toward the load 21 using capillarity, and is configured with, for example, glass fiber, a porous ceramic, or the like.

The load 21 atomizes the aerosol source 22 without combustion by power which is supplied from the power supply 12 through the discharging terminal 41. The load 21 is configured with a heating wire wound with a predetermined pitch (a coil). However, the load 21 needs only to be an element capable of atomizing the aerosol source 22, thereby generating an aerosol, and is, for example, a heating element or an ultrasonic wave generator. Examples of the heating element include a heating resistor, a ceramic heater, an induction heating type heater, and so on.

The aerosol channel 25 is provided on the downstream side of the load 21 on the center line L of the power supply unit 10.

The end cap 26 includes a cartridge storage part 26a for storing a part of the second cartridge 30, and a connecting passage 26b for connecting the aerosol channel 25 and the cartridge storage part 26a.

### (SECOND CARTRIDGE)

The second cartridge 30 holds a flavor source 31. The end part of the second cartridge 30 on the first cartridge (20) side is stored in the cartridge storage part 26a provided in the end cap 26 of the first cartridge 20, so as to be able to be removed. The end part of the second cartridge 30 on the opposite side to the first cartridge (20) side is configured as an inhalation port 32 for the user. However, the inhalation port 32 does not necessarily need to be configured integrally with the second cartridge 30 so as not to be separable from the second cartridge, and may be configured to be able to be attached to and detached from the second cartridge 30. If the inhalation port 32 is configured separately from the power supply unit 10 and the first cartridge 20 as described above, it is possible to keep the inhalation port 32 sanitary.

The second cartridge 30 adds a flavor to the aerosol generated by atomizing the aerosol source 22 by the load 21, by passing the aerosol through the flavor source 31. As a raw material piece which constitutes the flavor source, a compact made by forming shredded tobacco or a tobacco raw material into a grain shape can be used. The flavor source 31 may be configured with a plant (such as mint or a herbal medicine, or a herb) other than tobacco. To the flavor source 31, a flavoring agent such as menthol may be added.

The aerosol inhaler 1 of the present embodiment can generate an aerosol containing the flavor by the aerosol source 22, the flavor source 31, and the load 21. In other words, the aerosol source 22 and the flavor source 31 can be referred to as an aerosol generation source for generating an aerosol.

The configuration of the aerosol generation source which can be used in the aerosol inhaler 1 is not limited to the configuration in which the aerosol source 22 and the flavor source 31 are configured separately, and may be a configuration in which the aerosol source 22 and the flavor source 31 are formed integrally, a configuration in which the flavor source 31 is omitted and the aerosol source 22 contains a substance which can be contained in the flavor source 31, a configuration in which the aerosol source 22 contains a medical substance or the like instead of the flavor source 31, or the like.

In the aerosol inhaler 1 configured as described above, as shown by an arrow B in Fig. 3, air entering from the intake (not shown in the drawings) formed in the power supply unit case 11 passes through the air supply part 42, and passes near the load 21 of the first cartridge 20. The load 21 atomizes the aerosol source 22 drawn from the reservoir 23 by the wick 24. The aerosol generated by atomizing flows through the aerosol channel 25 together with the air entering from the intake, and is supplied to the second cartridge 30 through the connecting passage 26b. The aerosol supplied to the second cartridge 30 passes through the flavor source 31, whereby the flavor is added, and is supplied to the inhalation port 32.

Also, in the aerosol inhaler 1, an notifying unit 45 for notifying a variety of information is provided (see Fig. 5). The notifying unit 45 may be configured with a light emitting element, or may be configured with a vibrating element, or may be configured with a sound output element. Alternatively, the notifying unit 45 may be a combination of two or more elements of light emitting elements, vibrating elements, and sound output elements. The notifying unit 45 may be provided in any one of the power supply unit 10, the first cartridge 20, and the second cartridge 30; however, it is preferable that the notifying unit be provided in the power supply unit 10. For example, the area around the operation unit 14 is configured to have translucency to permit light which is emitted by a light emitting element such as an LED to pass through.

### (ELECTRIC CIRCUIT)

Now, the electric circuit of the power supply unit 10 will be described with reference to Fig. 6.

The power supply unit 10 includes the power supply 12, a positive electrode side discharging terminal 41a and a negative electrode side discharging terminal 41b which constitute the discharging terminal 41, a positive electrode side charging terminal 43a and a negative electrode side charging terminal 43b which constitute the charging terminal 43, the control unit 50 which is connected between the positive electrode side of the power supply 12 and the positive electrode side discharging terminal 41a and between the negative electrode side of the power supply 12 and the negative electrode side discharging terminal 41b, the voltage sensor 16 which measures the voltage of the power supply 12, the charger 13 which is disposed on the power transmission path between the charging terminal 43 and the power supply 12, and a switch 19 which is disposed on the power transmission path between the power supply 12 and the discharging terminal 41. The switch 19 is configured with, for example, a MOSFET, and is opened and closed by control of the control unit 50 on the gate voltage. The control unit 50 can determine that the external power supply 60 is connected to the charging terminal 43, for example, on the basis of a variation in small current flowing in the control unit 50.

In the electric circuit diagram of the power supply unit 10 shown in Fig. 6, the control unit 50 and the voltage sensor 16 are separate parts. Alternatively, the control unit 50 may have the function of measuring the voltage of the power supply 12. Also, in the electric circuit of the power supply unit 10 shown in Fig. 6, the switch 19 is provided between the positive electrode side of the power supply 12 and the positive electrode side discharging terminal 41a. Instead of this so-called plus control type, the switch 19 may be a minus control type which is provided between the negative electrode side discharging terminal 41b and the negative electrode side of the power supply 12.

### (CONTROL UNIT)

Now, the configuration of the control unit 50 will be described in more detail.

As shown in Fig. 5, the control unit 50 includes an aerosol generation request detecting unit 51, an operation detecting unit 52, a power control unit 53, and a notification control unit 54.

The aerosol generation request detecting unit 51 detects a request for aerosol generation on the basis of the output result of the inhalation sensor 15. The inhalation sensor 15 is configured to output the value of a variation in the pressure in the power supply unit 10 caused by inhalation of the user through the inhalation port 32. The inhalation sensor 15 is, for example, a pressure sensor for outputting an output value (for example, a voltage value or a current value) according to atmospheric pressure which varies according to the flow of air which is sucked from the intake (not shown in the drawings) toward the inhalation port 32 (i.e. a puff action of the user).

The operation detecting unit 52 detects an operation which is performed on the operation unit 14 by the user.

The notification control unit 54 controls the notifying unit 45 such that the notifying unit notifies a variety of information. For example, the notification control unit 54 controls the notifying unit 45 in response to detection of a timing to replace the second cartridge 30, such that the notifying unit notifies the timing to replace the second cartridge 30. The notification control unit 54 notifies a timing to replace the second cartridge 30, on the basis of the number of puff actions and the cumulative time for which power has been supplied to the load 21, stored in the memory 18. The notification control unit 54 is not limited to notification of a timing to replace the second cartridge 30, and may notify a timing to replace the first cartridge 20, a timing to replace the power supply 12, a timing to charge the power supply 12, and so on.

The power control unit 53 controls discharging of the power supply 12 through the discharging terminal 41 by switching on and off the switch 19 if the aerosol generation request detecting unit 51 detects the request for aerosol generation.

The power control unit 53 performs control such that the amount of aerosol which is generated by atomizing the aerosol source by the load 21 falls in a desired range, i.e. such that the amount of power which is supplied from the power supply 12 to the load 21 falls in a predetermined range. Specifically, the power control unit 53 controls switching on and off of the switch 19 by, for example, PWM (Pulse Width Modulation) control. Alternatively, the power control unit 53 may control switching on and off of the switch 19 by PFM (Pulse Frequency Modulation) control.

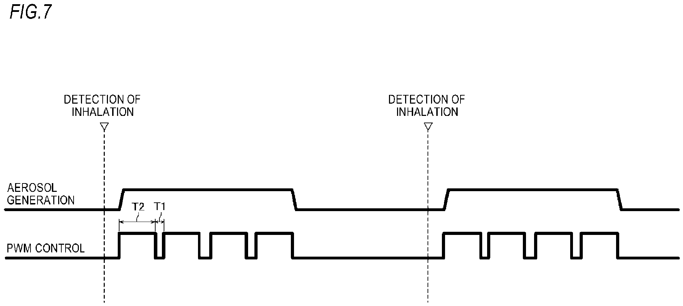

The power control unit 53 may stop supply of power from the power supply 12 to the load 21 if a predetermined period passes after start of supply of power to the load 21. In other words, even while the user is actually performing a puff action, if the puff period exceeds a certain period, the power control unit 53 stops supply of power from the power supply 12 to the load 21. The certain period is determined to suppress variation in user's puff period. The power control unit 53 controls the on/off duty ratio of the switch 19 for one puff action, according to the amount of power stored in the power supply 12. For example, as shown in Fig. 7, the power control unit 53 controls the interval (pulse interval T1) between ON times for which power is supplied from the power supply 12 to the load 21 and controls the length of each ON time (pulse width T2) for which power is supplied from the power supply 12 to the load 21.

Also, the power control unit 53 detects an electric connection between the charging terminal 43 and the external power supply 60, and controls charging of the power supply 12 through the charging terminal 43.

Here, in the power supply 12 which is used in the aerosol inhaler 1, if charging and discharging are performed at the same time, deterioration of the power supply 12 may accelerate. If charging and discharging are performed at the same time, the temperature of the power supply 12 is likely to rise. For this reason, the structure and composition in the power supply 12 may change, and deterioration of the power supply 12 may accelerate. Especially, considering that the aerosol inhaler 1 is made similar to traditional cigarettes in shape and weight, it is difficult to mount a temperature controller for the power supply 12 as compared to other electronic devices. Therefore, in the aerosol inhaler 1, it is preferable not to perform discharging and charging at the same time in order to manage the temperature of the power supply 12. Especially, in the case of using a lithium-ion secondary battery in which safe management is important, the necessity of temperature management increases.

As described above, the aerosol inhaler 1 is configured such that in the state where discharging of the power supply 12 through the discharging terminal 41 is possible, the external power supply 60 can be electrically connected to the charging terminal 43. In other words, the aerosol inhaler 1 is configured to be structurally able to perform charging and discharging of the power supply 12 at the same time.

For this reason, in the state where the charging terminal 43 and the external power supply 60 are electrically connected, in the case of detecting the aerosol generation request, the power control unit 53 controls the charger 13 and the switch 19 by charge/discharge control to be described below, thereby prohibiting charging and discharging of the power supply 12 from be performed at the same time. However, some types of charge/discharge control to be described below may be configured as programs which can execute them, and can be read into the power supply unit 10 and be executed by the power supply unit 10.

### (CHARGE/DISCHARGE CONTROL)

### <FIRST EXAMPLE>

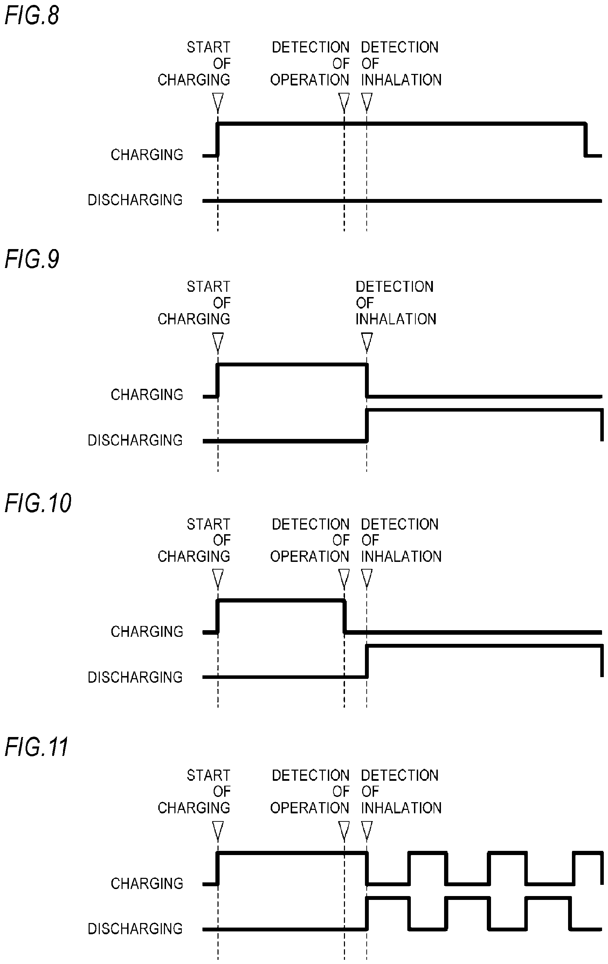

According to charge/discharge control of a first example, as shown in Fig. 8, in the state where the external power supply 60 is connected to the charging terminal 43, even if the operation unit 14 is operated, the operation on the operation unit 14 is invalidated, and even if the aerosol generation request detecting unit 51 detects the aerosol generation request, the power control unit 53 prohibits discharging of the power supply 12 through the discharging terminal 41. In other words, in the case where the external power supply 60 and the charging terminal 43 are electrically connected, and the aerosol generation request is detected, the power control unit 53 performs only charging. This charge/discharge control ends when charging is completed, or when the external power supply 60 is removed from the charging terminal 43.

In the state where the external power supply 60 is connected to the charging terminal 43, instead of invalidating an operation performed on the operation unit 14 and detection of inhalation by the inhalation sensor 15, the power supply of the operation unit 14 and the inhalation sensor 15 may be turned off. In this case, it is possible to surely prevent the aerosol generation request detecting unit 51 and the operation detecting unit 52 from functioning.

It is preferable that the power control unit 53 suppress discharging immediately after completion of charging. In general, the internal resistance of the power supply 12 during charging is higher than the internal resistance of the power supply 12 during discharging. Therefore, the temperature of the power supply 12 is likely to be higher during discharging than during charging. Since the temperature of the power supply 12 is high immediately after completion of charging, by suppressing aerosol generation immediately after completion of charging, it is possible to suppress a further rise in the temperature of the power supply.

Also, the power control unit 53 may require inhalation and an operation other than the inhalation in order to generate an aerosol immediately after completion of charging. In order to generate an aerosol immediately after completion of charging of the power supply 12, by requiring not only inhalation but also an operation other than inhalation (for example, an operation on the operation unit 14), it is possible to secure a time required until discharging starts.

According to the charge/discharge control of the first example, in the state where the charging terminal 43 and the external power supply 60 are electrically connected, in the case of detecting the aerosol generation request, the power control unit 53 performs only charging, thereby surely prohibiting aerosol generation such that charging and discharging of the power supply 12 are not performed at the same time. Also, by performing only charging, it is possible to actively charge the power supply 12.

### <SECOND EXAMPLE>

According to charge/discharge control of a second example, as shown in Fig. 9, in the state where the external power supply 60 is connected to the charging terminal 43, in the case where the aerosol generation request detecting unit 51 detects the aerosol generation request, charging of the power supply 12 through the charging terminal 43 is stopped. In other words, the power control unit 53 stops charging of the power supply 12 through the charging terminal 43 in response to the aerosol generation request. However, it should be noted that the aerosol generation request is an operation which can be performed while the electric connection between the charging terminal 43 and the external power supply 60 is maintained.

Also, the power control unit 53 starts discharging of the power supply 12 through the discharging terminal 41 after the stop of charging. In other words, in the case where the external power supply 60 and the charging terminal 43 are electrically connected, and the aerosol generation request is detected, the power control unit 53 performs only discharging. In this charge/discharge control, when inhalation ends, or a predetermined period elapses, i.e. after discharging stops, charging may be restarted. Also, in discharging of the power supply 12 through the discharging terminal 41 shown in Fig. 9, during the discharging period, the ON state is maintained, and can be controlled by PWM control as described above (the same is true for charge/discharge control of a third example shown in Fig. 10).

According to the charge/discharge control of the second example, by stopping charging in response to user's inhalation, it is possible to perform control such that charging and discharging of the power supply 12 are not performed at the same time. Also, in the case where the external power supply 60 and the charging terminal 43 are electrically connected, and the aerosol generation request is detected, only discharging is performed. Therefore, it is possible to satisfy the user's request. Also, after stop of discharging, charging is restarted. Therefore, it is possible to actively charging while performing control such that charging and discharging of the power supply 12 are not performed at the same time.

### <THIRD EXAMPLE>

According to the charge/discharge control of the third example, as shown in Fig. 10, in the state where the external power supply 60 is connected to the charging terminal 43, in the case where the operation detecting unit 52 detects an operation performed on the operation unit 14, charging of the power supply 12 through the charging terminal 43 is stopped. In other words, the power control unit 53 stops charging of the power supply 12 through the charging terminal 43 in response to the operation on the operation unit 14. However, an operation which is required to stop charging of the power supply 12 is not limited to an operation on the operation unit 14, and needs only to be an operation which can be performed while the electric connection between the charging terminal 43 and the external power supply 60 is maintained.

Also, in this state, if the aerosol generation request detecting unit 51 detects the aerosol generation request, the power control unit 53 starts discharging of the power supply 12 through the discharging terminal 41. In other words, in order to generate an aerosol after the stop of charging of the power supply 12, the power control unit 53 requires an operation (user's inhalation) other than the operation (the operation on the operation unit 14) which was the trigger for the stop of charging of the power supply 12. In this charge/discharge control, when inhalation ends, or a predetermined period elapses, i.e. after discharging stops, charging may be restarted.

However, in the course of charging or when charging is restarted, the power supply of the inhalation sensor 15 may be turned off. In this case, it is possible to suppress the amount of power which is stored in the power supply 12 while surely restraining charging and discharging from being performed at the same time.

According to the charge/discharge control of the third example, in order to generate an aerosol after the stop of charging of the power supply 12, the operation (user's inhalation) other than the operation (the operation on the operation unit 14) which was the trigger for the stop of charging of the power supply 12 is required. Therefore, while it is possible to restrain charging and discharging of the power supply 12 from being performed at the same time, it is possible to prevent aerosol generation from being caused by an erroneous operation on the operation unit 14. Also, after stop of discharging, charging is restarted. Therefore, it is possible to actively perform charging while performing control such that charging and discharging of the power supply 12 are not performed at the same time.

### <FOURTH EXAMPLE>

According to charge/discharge control of a fourth example, as shown in Fig. 11, in the state where the external power supply 60 is connected to the charging terminal 43, in the case where the aerosol generation request detecting unit 51 detects the aerosol generation request, discharging and charging are alternately performed. In other words, in PWM control, the power control unit 53 stops charging when the switch 19 is on, and performs charging when the switch 19 is off.

According to the charge/discharge control of the fourth example, in the case where the external power supply 60 and the charging terminal 43 are electrically connected, and the aerosol generation request is detected, discharging and charging are alternately performed, whereby while it is possible to perform control such that charging and discharging of the power supply 12 are not performed at the same time, it is possible to satisfy charging of the power supply 12 and the user's request at the same time.

### <FIFTH EXAMPLE>

In the types of charge/discharge control of the second to fourth examples, in order to switch from charging to discharging, user's inhalation and/or an operation on the operation unit 14 is required; however, even in order to switch from discharging to charging, the predetermined operation may be required. The predetermined operation is an operation on the operation unit 14, such as pressing the operation unit 14, or pressing and holding down the operation unit 14. Since the predetermined operation is required in order to switch from charging to discharging and/or in order to switch from discharging to charging, it is possible to prevent charging and discharging of the power supply 12 from being performed at the same time, and it is possible to suppress deterioration of the power supply 12. The predetermined operation is not limited to the operation on the operation unit 14, and needs only to be an operation which can be performed while the electric connection between the charging terminal 43 and the external power supply 60 is maintained.

### <SIXTH EXAMPLE>

In order to switch from charging to discharging, instead of user's inhalation and/or the predetermined operation which can be performed while the electric connection between the charging terminal 43 and the external power supply 60 is maintained, elapse of a predetermined time may be required. Also, even in order to switch from discharging to charging, elapse of the predetermined time may be required. By requiring elapse of the predetermined time in order to switch from charging to discharging and/or in order to switch from discharging to charging, it is possible to prevent charging and discharging of the power supply 12 from being performed at the same time, and it is possible to suppress deterioration of the power supply 12. The predetermined time can be measured by a timer.

### <MODIFICATIONS>

In the types of charge/discharge control of the second to fourth examples, in the case where the external power supply 60 and the charging terminal 43 are electrically connected, and the aerosol generation request is detected, power may be discharged from the power supply 12 to the load 21. In this case, power may be discharged from the external power supply 60 directly to the load 21.

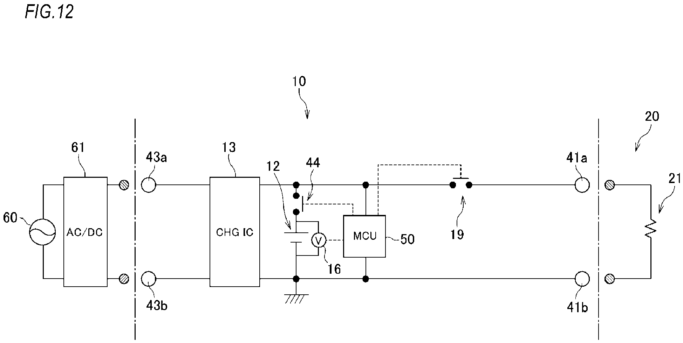

In order to make it possible to discharge power from the external power supply 60 directly to the load 21 through the discharging terminal 41, as shown in Fig. 12, a switch 44 for shutting off the power supply 12 may be provided in the circuit for electrically connecting the external power supply 60 and the load 21. If the switch 44 is turned off and the switch 19 is turned on, it becomes possible to discharge power from the external power supply 60 to the load 21. Therefore, it is possible to avoid power consumption of the power supply 12.

However, the present invention is not limited to the above-described embodiment, and modifications, improvements, etc. can be made properly.

For example, the number of operation units 14 is not limited to one, and may be two or more, and an operation unit to be used to activate and shut off the control unit 50 and an operation unit to be operated in order to switch from charging to discharging or in order to switch from discharging to charging may be different from each other.

In this specification, at least the following inventions (1) to (22) are disclosed.
(1) A power supply unit for an aerosol inhaler comprising:
   a power supply unit;
   a charging terminal that is able to be electrically connected to an external power supply capable of charging the power supply;
   a discharging terminal that is able to be electrically connected to a load for generating an aerosol from an aerosol source and is separate from the charging terminal; and
   a control unit that is configured to detect a request for aerosol generation and controls discharging of the power supply through the discharging terminal and charging of the power supply through the charging terminal, wherein
   the power supply unit is configured such that the external power supply can be electrically connected to the charging terminal in a state where discharging of the power supply through the discharging terminal is possible, and
   the control unit performs control such that the charging and the discharging are not performed at the same time in a case where the control unit detects the request in a state where the charging terminal and the external power supply are electrically connected.

According to (1), in the case of detecting the aerosol generation request in the state where the charging terminal and the external power supply are electrically connected, the control unit performs control such that the charging and the discharging of the power supply are not performed at the same time, whereby it is possible to suppress deterioration of the power supply.

(2) The power supply unit according to (1), wherein
at least one of a USB terminal, a micro USB terminal, and a Lightning terminal can be connected to the charging terminal.

According to (2), since it is possible to connect at least one of the USB terminal, the micro USB terminal, and the Lightning terminal to the charging terminal, it is possible to charge the power supply from a versatile external power supply cable.

(3) The power supply unit according to (2), wherein
the power supply unit extends in a predetermined direction,
the discharging terminal is provided at one end in the predetermined direction, and
the charging terminal is provided on a side surface of the other end in the predetermined direction.

According to (3), since the charging terminal is provided on the side surface of the other end part on the opposite side to the discharging terminal, it is possible to keep the power supply unit in a standing posture during charging. Also, as compared to the case where the charging terminal is provided on the bottom surface, it is hard for the insertion/removal force of the external power supply cable to act. Therefore, it is possible to suppress damage of the charging terminal.

(4) The power supply unit according to (1), wherein
the charging terminal is a power receiving part able to receive power from the external power supply in a non-contact manner.

According to (4), since the charging terminal is a power receiving part able to receive power from the external power supply in a non-contact manner, it is possible to charge the power supply without inserting and removing any external power supply cable. Therefore, it is possible to improve user convenience.

(5) The power supply unit according to any one of (1) to (4), wherein
the control unit is configured to suppress the discharging immediately after completion of the charging.

According to (5), since the temperature of the power supply is high immediately after completion of charging, by suppressing aerosol generation immediately after completion of the charging, it is possible to suppress a further rise in the temperature of the power supply.

(6) The power supply unit according to any one of (1) to (4), wherein
the control unit requires inhalation and an operation different from the inhalation in order to generate an aerosol immediately after completion of the charging.

According to (6), since the temperature of the power supply immediately after completion of charging is high, in order to generate an aerosol immediately after completion of the charging of the power supply, in addition to inhalation, the operation different from the inhalation is required, whereby, it is possible to secure a time until discharging starts.

(7) The power supply unit according to (5) or (6), further comprising:
a charger that is able to control charging power input from the charging terminal to the power supply.

According to (7), since the power supply unit includes the charger, it is possible to make a charger for the external power supply unnecessary. Also, since it is unnecessary to carry a dedicated charger, the chance of charging increases.

(8) The power supply unit according to any one of (1) to (7), wherein
the control unit stops the charging in response to detection of a predetermined operation which can be performed in a state where an electric connection between the charging terminal and the external power supply is maintained.

According to (8), the control unit stops the charging in response to detection of the predetermined operation which can be performed in the state where the electric connection between the charging terminal and the external power supply is maintained, thereby performing control such that the charging and the discharging of the power supply are not performed at the same time.

(9) The power supply unit according to (8), further comprising:
an operation unit that a user can operate,
wherein the predetermined operation is an operation on the operation unit.

According to (9), the control unit stops the charging in response to the operation on the operation unit, thereby performing control such that the charging and the discharging of the power supply are not performed at the same time.

(10) The power supply unit according to (9), wherein
the control unit requires an operation different from the predetermined operation in order to generate an aerosol after stopping the charging of the power supply.

According to (10), in order to generate the aerosol after stopping the charging of the power supply, the control unit requires the operation different from the predetermined operation, whereby, while it is possible to perform control such that the charging and the discharging of the power supply are not performed at the same time, it is possible to prevent aerosol generation from being caused by an erroneous operation on the operation unit.

(11) The power supply unit according to (9) or (10), wherein:
an operation on the operation unit preformed when the power supply unit is off activates the control unit.

According to (11), since the operation unit for activating the control unit is also used as the operation unit on which the operation to be a trigger for stopping the charging is performed, it is possible to prevent the power supply unit from becoming complicated. Also, it is possible to restrain the cost, weight, and size of the aerosol inhaler from increasing.

(12) The power supply unit according to (8), wherein
the predetermined operation is inhalation of a user.

According to (12), the control unit stops the charging in response to the inhalation of the user, thereby capable of performing control such that the charging and the discharging of the power supply are not performed at the same time.

(13) The power supply unit according to any one of (8) to (12), wherein
the control unit restarts the charging after stopping the discharging.

According to (13), since the control unit restarts the charging after stopping the discharging, it is possible to actively perform charging while performing control such that the charging and the discharging of the power supply are not performed at the same time.

(14) The power supply unit according to any one of (1) to (7), wherein
in a case where the external power supply and the charging terminal are electrically connected, and the control unit detects the request, the control unit performs only the charging.

According to (14), in the case where the external power supply and the charging terminal are electrically connected, and the control unit detects the aerosol generation request, the control unit performs only the charging, thereby capable of actively charging the power supply.

(15) The power supply unit according to any one of (1) to (7), wherein
in a case where the external power supply and the charging terminal are electrically connected, and the control unit detects the request, the control unit performs only the discharging.

According to (15), in the case where the external power supply and the charging terminal are electrically connected, and the control unit detects the aerosol generation request, the control unit performs only the discharging, thereby capable of satisfying the user's request.

(16) The power supply unit according to any one of (1) to (7), wherein:
in a case where the external power supply and the charging terminal are electrically connected, and the control unit detects the request, the control unit discharges power from the external power supply to the load through the discharging terminal.

According to (16), in the case where the external power supply and the charging terminal are electrically connected, and the control unit detects the aerosol generation request, the control unit discharges power from the external power supply to the load through the discharging terminal, thereby capable of avoiding power consumption of the power supply.

(17) The power supply unit according to any one of (1) to (7), wherein
in a case where the external power supply and the charging terminal are electrically connected, and the control unit detects the request, the control unit alternately performs the discharging and the charging.

According to (17), in the case where the external power supply and the charging terminal are electrically connected, and the control unit detects the aerosol generation request, the control unit alternately performs the discharging and the charging, thereby capable of satisfying the charging of the power supply and the user's request at the same time.

(18) A control method of a power supply unit for an aerosol inhaler, the power supply unit configured such that an external power supply can be electrically connected to a charging terminal in a state where discharging of a power supply through a discharging terminal is possible, the control method comprising:
prohibiting discharging of the power supply through the discharging terminal and charging of the power supply through the charging terminal from being performed at the same time in a case where a request for aerosol generation is detected in a state where the charging terminal and the external power supply are electrically connected.

According to (18), in the case where the aerosol generation request is detected in the state where the charging terminal and the external power supply are electrically connected, the charging and the discharging of the power supply are prohibited from being performed at the same time, whereby it is possible to suppress deterioration of the power supply.

(19) A control program which is for a power supply unit for an aerosol inhaler, the power supply unit configured such that an external power supply can be electrically connected to a charging terminal in a state where discharging of a power supply through a discharging terminal is possible, and which is for making a computer perform:
a control step of prohibiting discharging of the power supply through the discharging terminal and charging of the power supply through the charging terminal from being performed at the same time in a case where a request for aerosol generation is detected in a state where the charging terminal and the external power supply are electrically connected.

According to (19), in the case where the aerosol generation request is detected in the state where the charging terminal and the external power supply are electrically connected, the charging and the discharging of the power supply are prohibited from being performed at the same time, whereby it is possible to suppress deterioration of the power supply.

(20) A power supply unit for an aerosol inhaler, the power supply unit comprising:
a power supply unit;
a charging terminal that is able to be electrically connected to an external power supply capable of charging the power supply;
a discharging terminal that is able to be electrically connected to a load for generating an aerosol from an aerosol source and is separate from the charging terminal; and
a control unit that is configured to detect a request for aerosol generation and controls discharging of the power supply through the discharging terminal and charging of the power supply through the charging terminal, wherein
the power supply unit is configured such that the external power supply can be electrically connected to the charging terminal in a state where discharging of the power supply through the discharging terminal is possible, and
the control unit requires elapse of a predetermined time or a predetermined operation which can be performed in a state where an electric connection between the charging terminal and the external power supply is maintained in order to switch from the charging to the discharging and/or in order to switch from the discharging to the charging.

According to (20), in order to switch from the charging to the discharging or in order to switch from the discharging to the charging, the control unit requires elapse of the predetermined time or the predetermined operation which can be performed in the state where the electric connection between the charging terminal and the external power supply is maintained. In this way, it is possible to prevent the charging and the discharging of the power supply from being performed at the same time, and it is possible to suppress deterioration of the power supply.

(21) A control method of a power supply unit for an aerosol inhaler, the power supply unit configured such that an external power supply can be electrically connected to a charging terminal in a state where discharging of a power supply through a discharging terminal is possible, the control method comprising:
requiring elapse of a predetermined time or a predetermined operation which can be performed in a state where an electric connection between the charging terminal and the external power supply is maintained in order to switch from charging of the power supply through the charging terminal to discharging of the power supply through the discharging terminal and/or in order to switch from the discharging to the charging.

According to (21), in order to switch from the charging to the discharging or in order to switch from the discharging to the charging, elapse of the predetermined time or the predetermined operation which can be performed in the state where the electric connection between the charging terminal and the external power supply is maintained is required. In this way, it is possible to prevent the charging and the discharging of the power supply from being performed at the same time, and it is possible to deterioration of the power supply.

(22) A control program which is for a power supply unit for an aerosol inhaler, the power supply unit configured such that an external power supply can be electrically connected to a charging terminal in a state where discharging of a power supply through a discharging terminal is possible, and which is for making a computer execute:
a control step of requiring elapse of a predetermined time or a predetermined operation which can be performed in a state where an electric connection between the charging terminal and the external power supply is maintained in order to switch from charging of the power supply through the charging terminal to discharging of the power supply through the discharging terminal and/or in order to switch from the discharging to the charging.

According to (22), in order to switch from the charging to the discharging or in order to switch from the discharging to the charging, the predetermined operation which can be performed in the state where the electric connection between the charging terminal and the external power supply is maintained is required. In this way, it is possible to prevent the charging and the discharging of the power supply from being performed at the same time, and it is possible to suppress deterioration of the power supply.

According to (1), and (18) to (22), in the case where the aerosol generation request is detected in the state where the charging terminal and the external power supply are electrically connected, the charging and the discharging of the power supply are prohibited from being performed at the same time, whereby it is possible to suppress deterioration of the power supply. Therefore, there is energy saving effect in which it is possible to use the power supply for a long time without replacing with a brand new one.

According to the present invention, in a power supply unit for an aerosol inhaler which is structurally able to perform charging and discharging of a power supply at the same time, it is possible to perform control such that the charging and the discharging of the power supply are not performed at the same time, thereby capable of suppressing deterioration of the power supply.

Further embodiments are defined as E1 to E11, as follows:
E1. A power supply unit for an aerosol inhaler, the power supply unit comprising:
   a power supply unit;
   a charging terminal that is able to be electrically connected to an external power supply capable of charging the power supply;
   a discharging terminal that is able to be electrically connected to a load for generating an aerosol from an aerosol source and is separate from the charging terminal; and
   a control unit that is configured to detect a request for aerosol generation and controls discharging of the power supply through the discharging terminal and charging of the power supply through the charging terminal, wherein
   the power supply unit is configured such that the external power supply can be electrically connected to the charging terminal in a state where discharging of the power supply through the discharging terminal is possible, and
   the control unit performs control such that the charging and the discharging are not performed at the same time in a case where the control unit detects the request in a state where the charging terminal and the external power supply are electrically connected.
E2. The power supply unit according to embodiment 1, wherein
   at least one of a USB terminal, a micro USB terminal, and a Lightning terminal can be connected to the charging terminal.
E3. The power supply unit according to embodiment 2, wherein
   the power supply unit extends in a predetermined direction,
   the discharging terminal is provided at one end in the predetermined direction, and
   the charging terminal is provided on a side surface of the other end in the predetermined direction.
E4. The power supply unit according to embodiment 1, wherein
   the charging terminal is a power receiving part able to receive power from the external power supply in a non-contact manner.
E5. The power supply unit according to any one of embodiments 1 to 4, wherein
   the control unit is configured to suppress the discharging immediately after completion of the charging, or
   the control unit requires inhalation and an operation different from the inhalation in order to generate an aerosol immediately after completion of the charging.
E6. The power supply unit according to embodiment 5, further comprising:
   a charger that is able to control charging power input from the charging terminal to the power supply.
E7. The power supply unit according to any one of embodiments 1 to 6, wherein
   the control unit stops the charging in response to detection of a predetermined operation which can be performed in a state where an electric connection between the charging terminal and the external power supply is maintained.
E8. The power supply unit according to embodiment 7, further comprising:
   an operation unit that a user can operate,
   wherein the predetermined operation is an operation on the operation unit,
   preferably, an operation on the operation unit performed when the power supply unit is off activates the control unit.
E9. The power supply unit according to embodiment 8, wherein,
   the control unit requires an operation different from the predetermined operation in order to generate an aerosol after stopping the charging of the power supply,
   preferably, an operation on the operation unit performed when the power supply unit is off activates the control unit.
E10. The power supply unit according to any one of embodiments 7 to 9, wherein
   the control unit restarts the charging after stopping the discharging.
E11. A control method of a power supply unit for an aerosol inhaler, the power supply unit configured such that an external power supply can be electrically connected to a charging terminal in a state where discharging of a power supply through a discharging terminal is possible, the control method comprising:
   prohibiting discharging of the power supply through the discharging terminal and charging of the power supply through the charging terminal from being performed at the same time in a case where a request for aerosol generation is detected in a state where the charging terminal and the external power supply are electrically connected.

## Claims

1. A control method of a power supply unit for an aerosol inhaler, the power supply unit that is configured such that an external power supply can be electrically connected to a charging terminal in a state where discharging of a power supply through a discharging terminal is possible, the control method comprising:
in a case where the charging terminal and the external power supply are electrically connected and charging by the external power supply through the charging terminal is performed, performing only the charging among the charging and the discharging by preventing an inhalation sensor from detecting inhalation or invalidating detection of inhalation by an inhalation sensor.

2. The control method according to claim 1, further comprising:
suppressing the discharging after completion of the charging.

3. The control method according to claim 1 or 2, further comprising:
requiring the inhalation and an operation different from the inhalation in order to generate an aerosol after completion of the charging.

4. The control method according to any one of claims 1 to 3, further comprising:
stopping the charging in response to detection of a predetermined operation which can be performed in a state where an electric connection between the charging terminal and the external power supply is maintained.

5. The control method according to claim 4, wherein
the predetermined operation is an operation on an operation unit of the power supply unit.

6. The control method according to claim 5, further comprising:
requiring an operation different from the predetermined operation in order to generate an aerosol after stopping the charging of the power supply.

7. The control method according to claim 5 or 6, wherein
an operation on the operation unit performed when the power supply unit is off activates a control unit of the power supply unit.

8. The control method according to any one of claims 4 to 7, further comprising:
restarting the charging after stopping the discharging.

9. The control method according to any one of claims 1 to 8, further comprising:
in order to switch from the charging to the discharging, requiring elapse of a predetermined time, or a predetermined operation which can be performed in a state where an electric connection between the charging terminal and the external power supply is maintained.

10. A control program that causes a power supply unit for an aerosol inhaler to execute a process, the power supply unit that being configured such that an external power supply can be electrically connected to a charging terminal in a state where discharging of a power supply through a discharging terminal is possible, the process comprising:
in a case where the charging terminal and the external power supply are electrically connected and charging by the external power supply through the charging terminal is performed, performing only the charging among the charging and the discharging by preventing an inhalation sensor from detecting inhalation or invalidating detection of inhalation by an inhalation sensor.

11. The control program according to claim 10, wherein
the process further comprises suppressing the discharging after completion of the charging.

12. The control program according to claim 10 or 11, wherein
the process further comprises requiring the inhalation and an operation different from the inhalation in order to generate an aerosol after completion of the charging.

13. The control program according to any one of claims 10 to 12, wherein
the process further comprises stopping the charging in response to detection of a predetermined operation which can be performed in a state where an electric connection between the charging terminal and the external power supply is maintained.

14. The control program according to claim 13, wherein
the predetermined operation is an operation on an operation unit of the power supply unit.

15. The control program according to claim 14, wherein
the process further comprises requiring an operation different from the predetermined operation in order to generate an aerosol after stopping the charging of the power supply.

16. The control program according to claim 14 or 15, wherein
an operation on the operation unit performed when the power supply unit is off activates a control unit of the power supply unit.

17. The control program according to any one of claims 13 to 16, wherein
the process further comprises restarting the charging after stopping the discharging.

18. The control program according to any one of claims 10 to 17, wherein
the process further comprises, in order to switch from the charging to the discharging, requiring elapse of a predetermined time, or a predetermined operation which can be performed in a state where an electric connection between the charging terminal and the external power supply is maintained.
